# EUROPEAN PATENT APPLICATION

(11) **EP 3 922 262 A1**
(43) Date of publication of application: **15.12.2021**
(21) Application number: 20382495.8
(22) Date of filing: 08.06.2020
(51) Int. Cl.: A61K 39/215, C07K 14/005, C12Q 1/37

(54) **ASSAY FOR THE DETECTION OF THE CYS-LIKE PROTEASE (MPRO) OF SARS-COV-2**

(71) Applicant: Consejo Superior De Investigaciones Científicas (CSIC), 28006 Madrid (ES)
(72) Inventor: REYBURN, Hugh Thomson, 28049 Madrid (ES); VALÉS GÓMEZ, María del Mar, 28049 Madrid (ES); RODRÍGUEZ FRADE, José Miguel, 28049 Madrid (ES); CASASNOVAS SUELVES, José María, 28049 Madrid (ES)
(74) Representative: Hoffmann Eitle

(57) **Abstract**

In the present invention, we herein generally provide an *in vitro* method for detecting in at least one biological sample an antibody that binds to at least one epitope of the SARS-CoV-2 virus, comprising contacting said at least one biological sample with at least one isolated SARS-CoV-2 M^{pro} protein, or at least one fragment of said isolated SARS-CoV-2 M^{pro} protein comprising at least one epitope of the SARS-CoV-2 virus, and detecting the formation of an antigen-antibody complex between said virus protein or said fragment and an antibody present in said biological sample.

SEQUENCE LISTING

<110>  Consejo Superior de Investigaciones Cientificas (CSIC)

<120>  Assay for the detection of the Cys-like protease (Mpro) of
       SARS-CoV-2

<130>  905 286

<160>  10

<170>  PatentIn version 3.5

<210>  1
<211>  319
<212>  PRT
<213>  Artificial Sequence

<220>
<223>  Peptide sequence SARS-CoV-2 Mpro


<220>
<221>  PEPTIDE
<222>  (1)..(319)

<400>  1

Ser Ala Val Leu Gln Ser Gly Phe Arg Lys Met Ala Phe Pro Ser Gly
1               5                   10                  15


Lys Val Glu Gly Cys Met Val Gln Val Thr Cys Gly Thr Thr Thr Leu
            20                  25                  30


Asn Gly Leu Trp Leu Asp Asp Val Val Tyr Cys Pro Arg His Val Ile
        35                  40                  45


Cys Thr Ser Glu Asp Met Leu Asn Pro Asn Tyr Glu Asp Leu Leu Ile
    50                  55                  60


Arg Lys Ser Asn His Asn Phe Leu Val Gln Ala Gly Asn Val Gln Leu
65                  70                  75                  80


Arg Val Ile Gly His Ser Met Gln Asn Cys Val Leu Lys Leu Lys Val
                85                  90                  95


Asp Thr Ala Asn Pro Lys Thr Pro Lys Tyr Lys Phe Val Arg Ile Gln
            100                 105                 110


Pro Gly Gln Thr Phe Ser Val Leu Ala Cys Tyr Asn Gly Ser Pro Ser
        115                 120                 125


Gly Val Tyr Gln Cys Ala Met Arg Pro Asn Phe Thr Ile Lys Gly Ser
    130                 135                 140

Phe Leu Asn Gly Ser Cys Gly Ser Val Gly Phe Asn Ile Asp Tyr Asp
145                 150                 155                 160

Cys Val Ser Phe Cys Tyr Met His His Met Glu Leu Pro Thr Gly Val
                165                 170                 175

His Ala Gly Thr Asp Leu Glu Gly Asn Phe Tyr Gly Pro Phe Val Asp
            180                 185                 190

Arg Gln Thr Ala Gln Ala Ala Gly Thr Asp Thr Thr Ile Thr Val Asn
        195                 200                 205

Val Leu Ala Trp Leu Tyr Ala Ala Val Ile Asn Gly Asp Arg Trp Phe
    210                 215                 220

Leu Asn Arg Phe Thr Thr Thr Leu Asn Asp Phe Asn Leu Val Ala Met
225                 230                 235                 240

Lys Tyr Asn Tyr Glu Pro Leu Thr Gln Asp His Val Asp Ile Leu Gly
                245                 250                 255

Pro Leu Ser Ala Gln Thr Gly Ile Ala Val Leu Asp Met Cys Ala Ser
            260                 265                 270

Leu Lys Glu Leu Leu Gln Asn Gly Met Asn Gly Arg Thr Ile Leu Gly
        275                 280                 285

Ser Ala Leu Leu Glu Asp Glu Phe Thr Pro Phe Asp Val Val Arg Gln
    290                 295                 300

Cys Ser Gly Val Thr Phe Gln Leu Glu His His His His His His
305                 310                 315

<210>  2
<211>  960
<212>  DNA
<213>  Artificial Sequence

<220>
<223>  Nucleotide sequence of SARS-CoV-2 Mpro

<220>
<221>  misc_feature
<222>  (1)..(960)

<400>  2
tcagctgttt tgcagagtgg ttttagaaaa atggcattcc catctggtaa agttgagggt      60

tgtatggtac aagtaacttg tggtacaact acacttaacg gtctttggct tgatgacgta     120

gtttactgtc caagacatgt gatctgcacc tctgaagaca tgcttaaccc taattatgaa 180

gatttactca ttcgtaagtc taatcataat ttcttggtac aggctggtaa tgttcaactc 240

agggttattg gacattctat gcaaaattgt gtacttaagc ttaaggttga tacagccaat 300

cctaagacac ctaagtataa gtttgttcgc attcaaccag gacagacttt ttcagtgtta 360

gcttgttaca atggttcacc atctggtgtt taccaatgtg ctatgaggcc caatttcact 420

attaagggtt cattccttaa tggttcatgt ggtagtgttg gttttaacat agattatgac 480

tgtgtctctt tttgttacat gcaccatatg gaattaccaa ctggagttca tgctggcaca 540

gacttagaag gtaactttta tggacctttt gttgacaggc aaacagcaca agcagctggt 600

acggacacaa ctattacagt taatgtttta gcttggttgt acgctgctgt tataaatgga 660

gacaggtggt ttctcaatcg atttaccaca actcttaatg actttaacct tgtggctatg 720

aagtacaatt atgaacctct aacacaagac catgttgaca tactaggacc tctttctgct 780

caaactggaa ttgccgtttt agatatgtgt gcttcattaa aagaattact gcaaaatggt 840

atgaatggac gtaccatatt gggtagtgct ttattagaag atgaatttac accttttgat 900

gttgttagac aatgctcagg tgttactttc caactcgagc accaccacca ccaccactga 960

<210> 3
<211> 377
<212> PRT
<213> Artificial Sequence

<220>
<223> Peptide sequence SARS-CoV-2 nucleocapsid protein

<220>
<221> PEPTIDE
<222> (1)..(377)

<400> 3

Met Ala Ser Asp Asn Gly Pro Gln Asn Gln Arg Asn Ala Pro Arg Ile
1               5                   10                  15

Thr Phe Gly Gly Pro Ser Asp Ser Thr Gly Ser Asn Gln Asn Gly Glu
            20                  25                  30

Arg Ser Gly Ala Arg Ser Lys Gln Arg Arg Pro Gln Gly Leu Pro Asn
        35                  40                  45

Asn Thr Ala Ser Trp Phe Thr Ala Leu Thr Gln His Gly Lys Glu Asp
    50                  55                  60

Leu Lys Phe Pro Arg Gly Gln Gly Val Pro Ile Asn Thr Asn Ser Ser
65                  70                  75                  80

Pro Asp Asp Gln Ile Gly Tyr Tyr Arg Arg Ala Thr Arg Arg Ile Arg
                85                  90                  95

Gly Gly Asp Gly Lys Met Lys Asp Leu Ser Pro Arg Trp Tyr Phe Tyr
            100                 105                 110

Tyr Leu Gly Thr Gly Pro Glu Ala Gly Leu Pro Tyr Gly Ala Asn Lys
        115                 120                 125

Asp Gly Ile Ile Trp Val Ala Thr Glu Gly Ala Leu Asn Thr Pro Lys
    130                 135                 140

Asp His Ile Gly Thr Arg Asn Pro Ala Asn Asn Ala Ala Ile Val Leu
145                 150                 155                 160

Gln Leu Pro Gln Gly Thr Thr Leu Pro Lys Gly Phe Tyr Ala Glu Gly
                165                 170                 175

Ser Arg Gly Gly Ser Gln Ala Ser Ser Arg Ser Ser Ser Arg Ser Arg
            180                 185                 190

Asn Ser Ser Arg Asn Ser Thr Pro Gly Ser Ser Arg Gly Thr Ser Pro
        195                 200                 205

Ala Arg Met Ala Gly Asn Gly Gly Asp Ala Ala Leu Ala Leu Leu Leu
    210                 215                 220

Leu Asp Arg Leu Asn Gln Leu Glu Ser Lys Met Ser Gly Lys Gly Gln
225                 230                 235                 240

Gln Gln Gln Gly Gln Thr Val Thr Lys Lys Ser Ala Ala Glu Ala Ser
                245                 250                 255

Lys Lys Pro Arg Gln Lys Arg Thr Ala Thr Lys Ala Tyr Asn Val Thr
            260                 265                 270

Gln Ala Phe Gly Arg Arg Gly Pro Glu Gln Thr Gln Gly Asn Phe Gly
        275                 280                 285

Asp Gln Glu Leu Ile Arg Gln Gly Thr Asp Tyr Lys His Trp Pro Gln
    290                 295                 300

Ile Ala Gln Phe Ala Pro Ser Ala Ser Ala Phe Phe Gly Met Ser Arg
305                 310                 315                 320

Ile Gly Met Glu Val Thr Pro Ser Gly Thr Trp Leu Thr Tyr Thr Gly
                325                 330                 335

Ala Ile Lys Leu Asp Asp Lys Asp Pro Asn Phe Lys Asp Gln Val Ile
            340                 345                 350

Leu Leu Asn Lys His Ile Asp Ala Tyr Lys Thr Phe Pro Pro Thr Glu
        355                 360                 365

Pro Val Glu His His His His His His
    370                 375

<210>  4
<211>  1134
<212>  DNA
<213>  Artificial Sequence

<220>
<223>  Nucleotide sequence of SARS-CoV-2 nucleocapsid protein

<220>
<221>  misc_feature
<222>  (1)..(1134)

<400>  4
atggcttctg ataatggtcc gcaaaatcag cgtaatgcac cccgcattac gtttggtgga      60

ccctcagatt caactggcag taaccagaat ggagaacgca gtggggcgcg atcaaaacaa     120

cgtcggcccc aaggtttacc caataatact gcgtcttggt tcaccgctct cactcaacat     180

ggcaaggaag accttaaatt ccctcgagga caaggcgttc caattaacac caatagcagt     240

ccagatgacc aaattggcta ctaccgaaga gctaccagac gaattcgtgg tggtgacggt     300

aaaatgaaag atctcagtcc aagatggtat ttctactacc taggaactgg gccagaagct     360

ggacttccct atggtgctaa caaagacggc atcatatggg ttgcaactga gggagccttg     420

aatacaccaa aagatcacat tggcacccgc aatcctgcta acaatgctgc aatcgtgcta     480

caacttcctc aaggaacaac attgccaaaa ggcttctacg cagaagggag cagaggcggc     540

agtcaagcct cttctcgttc ctcatcacgt agtcgcaaca gttcaagaaa ttcaactcca     600

ggcagcagta ggggaacttc tcctgctaga atggctggca atggcggtga tgctgctctt     660

gctttgctgc tgcttgacag attgaaccag cttgagagca aaatgtctgg taaaggccaa     720

caacaacaag gccaaactgt cactaagaaa tctgctgctg aggcttctaa gaagcctcgg     780

caaaaacgta ctgccactaa agcatacaat gtaacacaag ctttcggcag acgtggtcca     840

gaacaaaccc aaggaaattt tggggaccag gaactaatca gacaaggaac tgattacaaa     900

cattggccgc aaattgcaca atttgccccc agcgcttcag cgttcttcgg aatgtcgcgc     960

attggcatgg aagtcacacc ttcgggaacg tggttgacct acacaggtgc catcaaattg    1020

gatgacaaag atccaaattt caaagatcaa gtcattttgc tgaataagca tattgacgca    1080

tacaaaacat tcccaccaac agagcctgtc gagcaccacc accaccacca ctga          1134

<210>  5
<211>  202
<212>  PRT
<213>  Artificial Sequence

<220>
<223>  Peptide sequence SARS-CoV-2 RBD

<220>
<221>  PEPTIDE
<222>  (1)..(202)

<400>  5

Gly Ser Arg Asn Leu Cys Pro Phe Gly Glu Val Phe Asn Ala Thr Arg
1               5                   10                  15

Phe Ala Ser Val Tyr Ala Trp Asn Arg Lys Arg Ile Ser Asn Cys Val
            20                  25                  30

Ala Asp Tyr Ser Val Leu Tyr Asn Ser Ala Ser Phe Ser Thr Phe Lys
        35                  40                  45

Cys Tyr Gly Val Ser Pro Thr Lys Leu Asn Asp Leu Cys Phe Thr Asn
    50                  55                  60

Val Tyr Ala Asp Ser Phe Val Ile Arg Gly Asp Glu Val Arg Gln Ile
65                  70                  75                  80

Ala Pro Gly Gln Thr Gly Lys Ile Ala Asp Tyr Asn Tyr Lys Leu Pro
                85                  90                  95

Asp Asp Phe Thr Gly Cys Val Ile Ala Trp Asn Ser Asn Asn Leu Asp
            100                 105                 110

Ser Lys Val Gly Gly Asn Tyr Asn Tyr Leu Tyr Arg Leu Phe Arg Lys
        115                 120                 125

Ser Asn Leu Lys Pro Phe Glu Arg Asp Ile Ser Thr Glu Ile Tyr Gln
    130                 135                 140

Ala Gly Ser Thr Pro Cys Asn Gly Val Glu Gly Phe Asn Cys Tyr Phe
145                 150                 155                 160

Pro Leu Gln Ser Tyr Gly Phe Gln Pro Thr Asn Gly Val Gly Tyr Gln
                165                 170                 175

Pro Tyr Arg Val Val Val Leu Ser Phe Glu Leu Leu His Ala Pro Ala
            180                 185                 190

Thr Val Cys Gly Pro Lys Leu Val Pro Arg
        195                 200

<210>  6
<211>  606
<212>  DNA
<213>  Artificial Sequence

<220>
<223>  Nucleotide sequence of SARS-CoV-2 RBD

<220>
<221>  misc_feature
<222>  (1)..(606)

<400>  6
ggatcccgca acttgtgccc ttttggtgaa gtttttaacg ccaccagatt tgcatctgtt      60

tatgcttgga acaggaagag aatcagcaac tgtgttgctg attattctgt cctatataat     120

tccgcatcat tttccacttt taagtgttat ggagtgtctc ctactaaatt aaatgatctc     180

tgctttacta atgtctatgc agattcattt gtaattagag gtgatgaagt cagacaaatc     240

gctccagggc aaactggaaa gattgctgat tataattata aattaccaga tgattttaca     300

ggctgcgtta tagcttggaa ttctaacaat cttgattcta aggttggtgg taattataat     360

tacctgtata gattgtttag gaagtctaat ctcaaacctt ttgagagaga tatttcaact     420

gaaatctatc aggccggtag cacaccttgt aatggtgttg aaggttttaa ttgttacttt     480

cctttacaat catatggttt ccaacccact aatggtgttg gttaccaacc atacagagta     540

gtagtacttt cttttgaact tctacatgca ccagcaactg tttgtggacc taaacttgtc     600

cctcgc                                                                606

<210>  7
<211>  34
<212>  DNA
<213>  Artificial Sequence

<220>
<223>  Oligo

<220>
<221>  misc_feature
<222>  (1)..(34)

<400>  7
gacccatggc ttcagctgtt tttcagagtg gttt                                  34

<210>  8

<211>  32
<212>  DNA
<213>  Artificial Sequence

<220>
<223>  Oligo.


<220>
<221>  misc_feature
<222>  (1)..(32)

<400>  8
gacctcgagt tggaaagtaa cacctgagca tt                                    32


<210>  9
<211>  44
<212>  DNA
<213>  Artificial Sequence

<220>
<223>  Oligo


<220>
<221>  misc_feature
<222>  (1)..(44)

<400>  9
gatccatggc ttctgataat ggtccgcaaa atcagcgtaa tgca                       44


<210>  10
<211>  29
<212>  DNA
<213>  Artificial Sequence

<220>
<223>  Oligo


<220>
<221>  misc_feature
<222>  (1)..(29)

<400>  10
caggtcgaca ggctctgttg gtgggaatg                                        29

## Description

### Technical field of the invention

The present invention relates to recombinantly expressed proteins from the SARS- CoV-2, in particular to the main protease (M^{pro}, also known as 3CLPro) of SARS-CoV-2, as well as fragments thereof, and their use in the detection in a biological sample of antibodies that bind to at least one epitope of the SARS-CoV-2 virus.

### Background of the invention

In December 2019, a new coronavirus (CoV) emerged in China to cause an acute respiratory disease known as coronavirus disease 19 (COVID-19). The virus was identified to be a betacoronavirus related to severe acute respiratory syndrome coronavirus (SARS-CoV) and thus, was named SARS-CoV-2. In less than two decades, this virus is the third known coronavirus to cross the species barrier and cause severe respiratory infections in humans following SARS-CoV in 2003 and Middle East respiratory syndrome in 2012, yet with unprecedented spread compared to the earlier two. Due to the rapid rise in number of cases and uncontrolled and vast worldwide spread, the WHO has declared SARS-CoV-2 a pandemic. As of March 14^{th} 2020, the virus had infected over 130,000 individuals in 122 countries, 3.7% of which had a fatal outcome. The rapid identification of the aetiology and the sharing of the genetic sequence of the virus, followed by international collaborative efforts initiated due to the emergence of SARS-CoV-2 have led to the rapid availability of real-time PCR diagnostic assays that support the case ascertainment and tracking of the outbreak. The availability of these has helped in patient detection and efforts to contain the virus. However, specific and validated serologic assays are still lacking at the moment and are urgently needed to understand the epidemiology of SARS-CoV-2.

Validated serologic assays are crucial for patient contact tracing, identifying the viral reservoir hosts and for epidemiological studies. Epidemiological studies are urgently needed to help uncover the burden of disease, in particular, the rate of asymptomatic infections, and to get better estimates on morbidity and mortality. Additionally, these epidemiological studies can help reveal the extent of virus spread in households, communities and specific settings; which could help guide control measures. Serological assays are also needed for evaluation of the results of vaccine trials and development of therapeutic antibodies. Among the four coronavirus structural proteins, the spike (S) and the nucleocapsid (N) are the main immunogens (Meyer B, Drosten C, Muller MA. Serological assays for emerging coronaviruses: challenges and pitfalls. Virus research. 2014 Dec 19;194:175-83). The development of serological assays for the detection of virus neutralizing antibodies and antibodies to the nucleocapsid (NP) protein and various spike (S) domains including the S1 subunit, and receptor binding domain (RBD) of SARS-CoV-2 in ELISA format have been described.

In the present invention we provide for further viral protein immunogens useful for such assays, that are not structural and are not incorporated into the infectious viral particle.

### Brief description of the figures

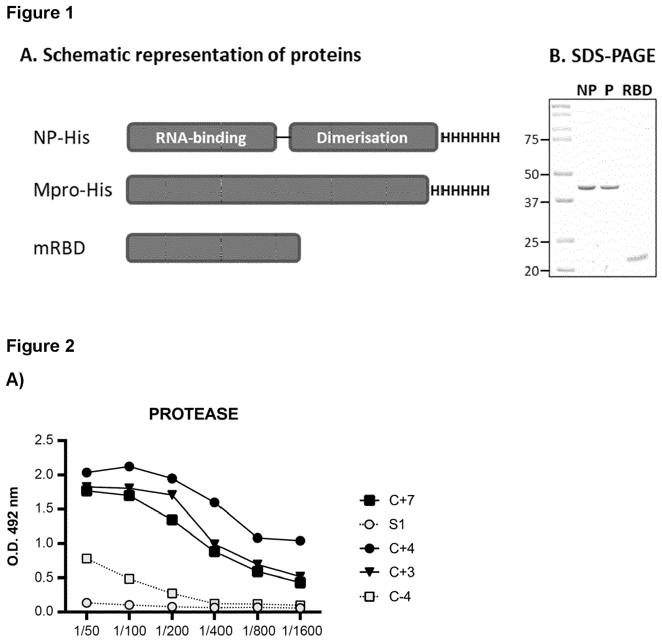

**Figure 1****.** SARS-CoV-2 protein purification. (A) Schematic representation of proteins expressed from the plasmid constructs. Cys-like protease (3CLpro, Mpro), nucleocapsid (NP) and mammalian (mRBD) (B) SDS-PAGE. After expression in the different systems, proteins were purified and fractions from gel filtration chromatography were run in SDS-PAGE.

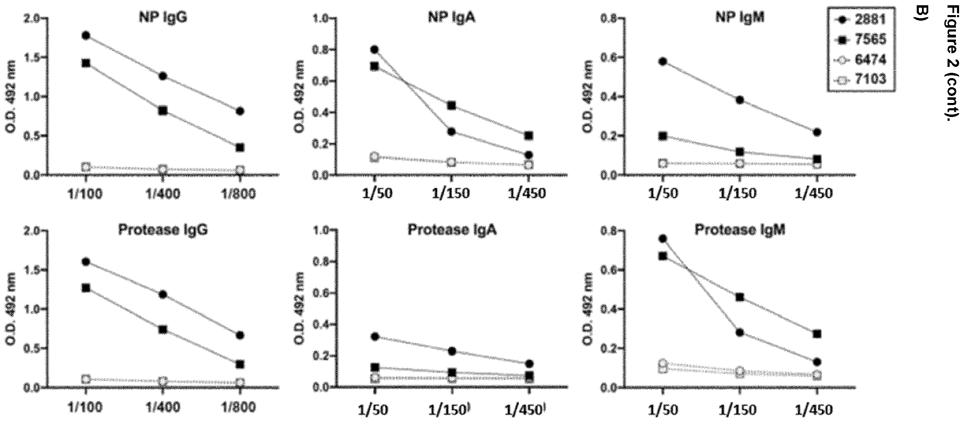

Figure 2. Detection of SARS-CoV-2 Mpro-specific antibodies by ELISA. (A) Sera titration on Mpro. Plates were coated with SARS-CoV-2 Mpro and sera dilutions (1/50 to 1/1600) were tested. Detection was performed using anti-human F(ab)2' antibody. (B) Isotype recognition. Plates coated with SARS-CoV-2 Mpro, and nucleoprotein (NP) were detected with antibodies directed against human Ig of the three different subclasses: IgG, IgA, IgM. Black symbols correspond to covid-19 patients and grey symbols to donor samples collected pre-covid-19.

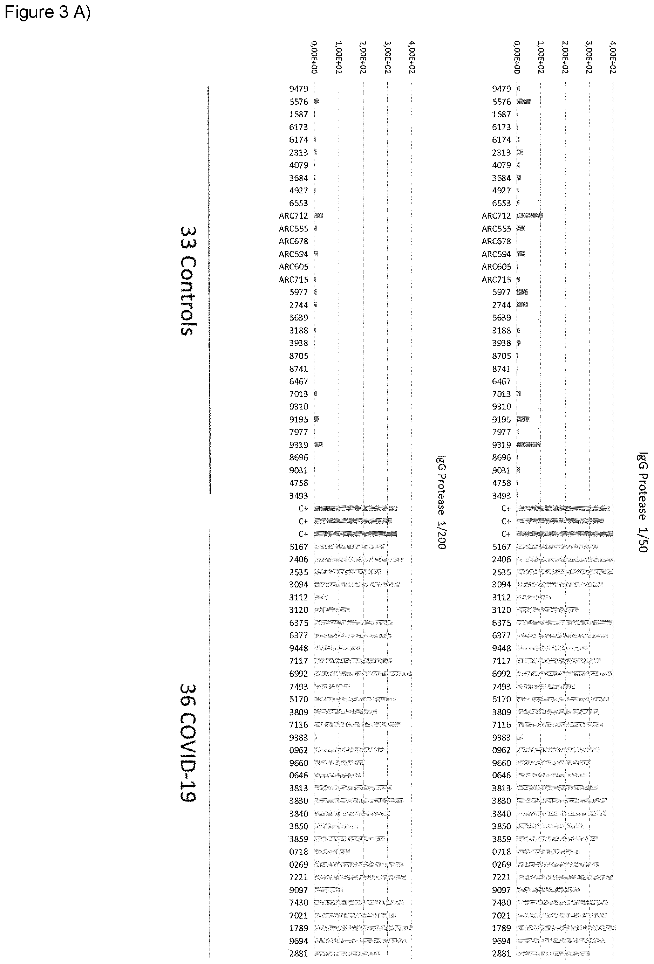

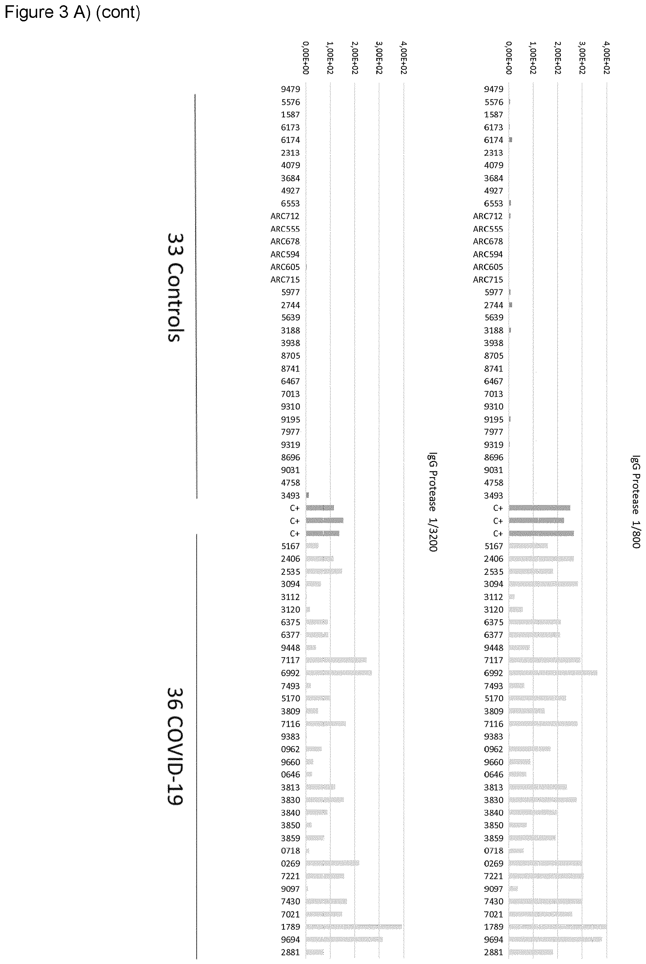

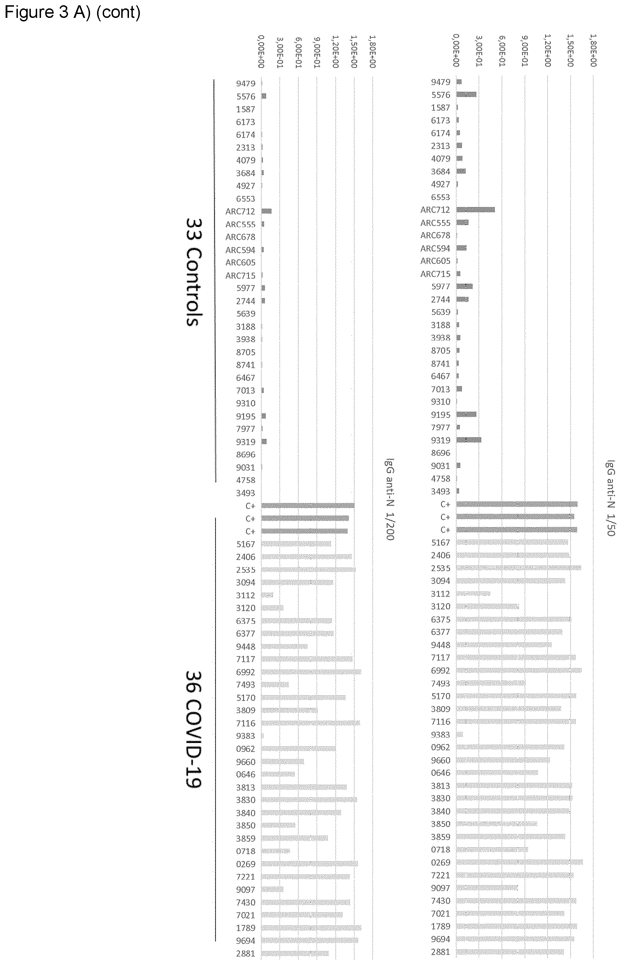

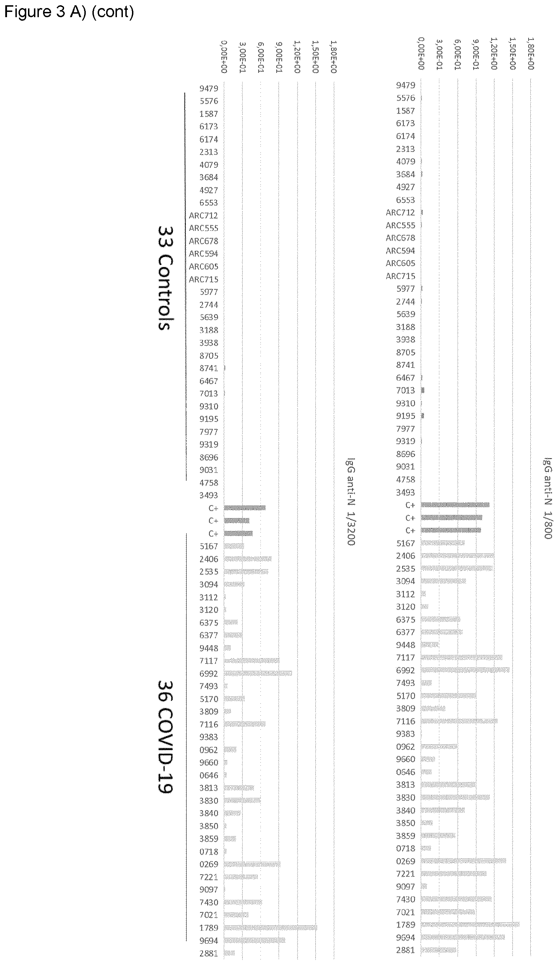

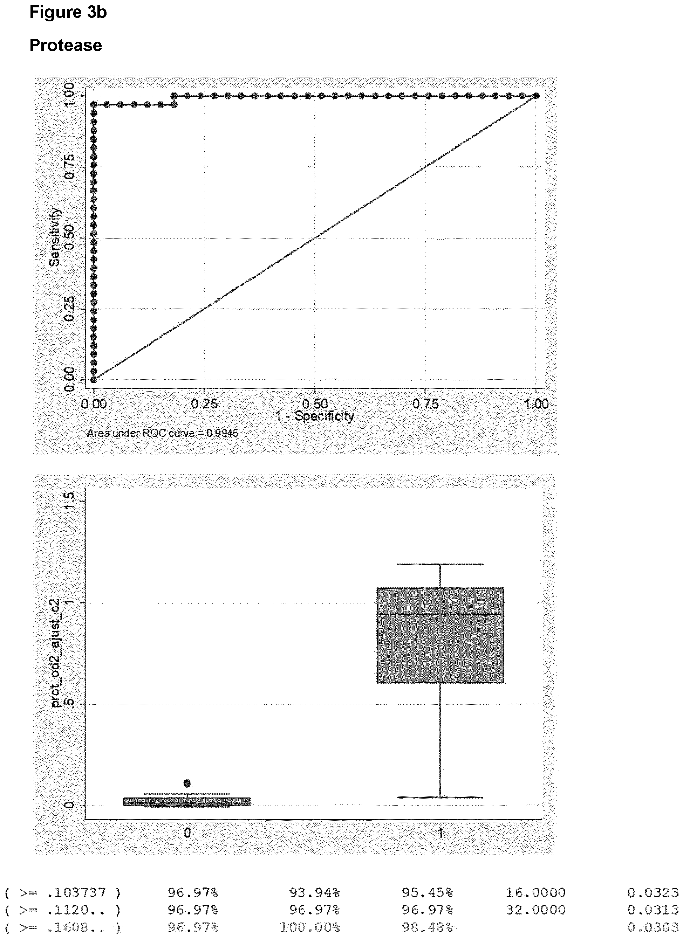

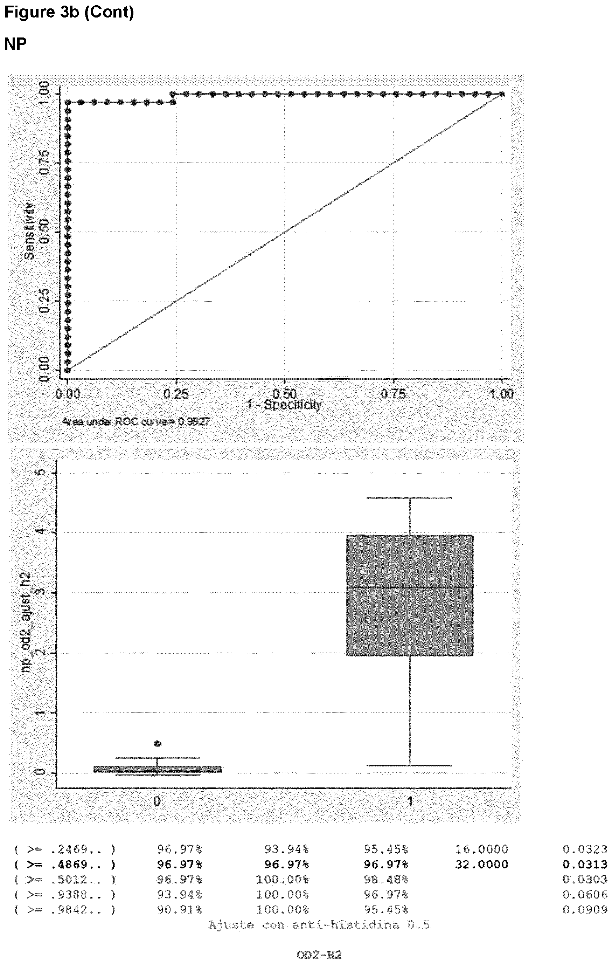

**Figure 3****: Comparative ELISA tests.** Plates coated with 0.5 ug/ml SARS-CoV-2 Mpro and nucleoprotein (NP) were used to perform the ELISA test on 36 COVID-19 positive (purple), 33 negative control. Detection was done using antibodies directed against human Ig of the three different subclasses: IgG, IgA, IgM. **A)** ELISA data of serum samples. **B)** ROC curves for the protease and NP assays of section A).

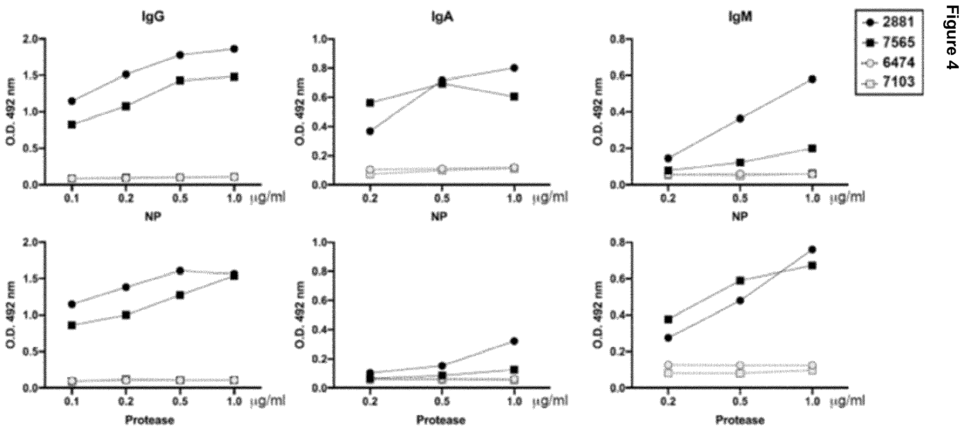

**Figure 4****.** Coating titration for detection of SARS-CoV-2-specific antibodies by ELISA. Plates were coated with SARS-CoV-2 Mpro and nucleoprotein (NP) and sera dilutions (1/50) to 1/1600) were tested. Detection was performed using antibodies directed against human Ig of the three different subclasses: IgG, IgA, IgM. Black symbols correspond to covid-19 patients and grey symbols to donors pre-covid-19.

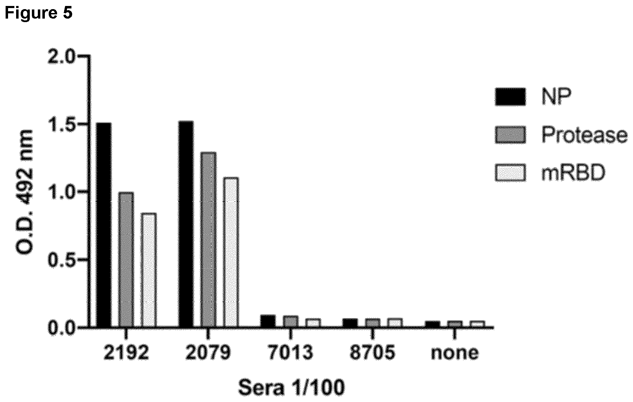

Figure 5: Comparison of Mpro and other viral antigens seroreactivity. Plates coated with either 0.5 ug/m SARS-CoV-2 Mpro or nucleoprotein (NP) or 1 µg/ml mRBD. serum samples diluted 1/100 were tested in ELISA assay and developed using anti-human IgG antibody. The two COVID19⁺ sera, negative sera and control wells are indicated.

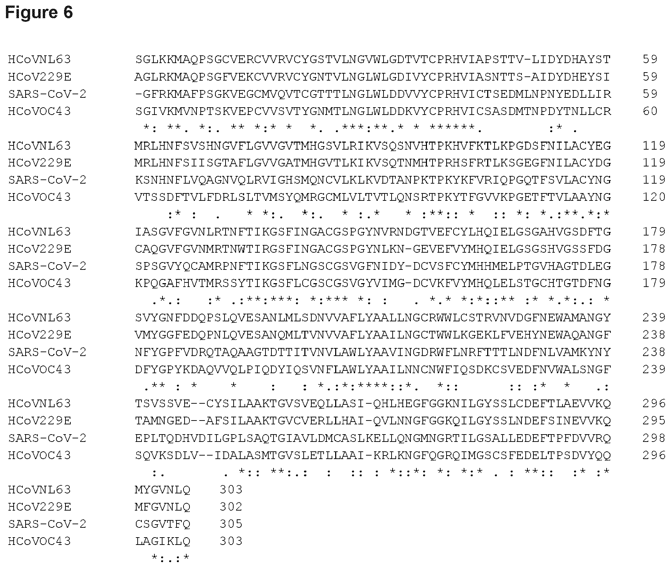

Figure 6. To explore the similarity between the Cys-like proteases of different coronaviruses, 3CLpro (Mpro) from SARS-CoV-2, HCovNL63 and HCov229E were aligned (Figure 7). The degree of similarity was around 40%.

### Description of the invention

The identification of the link between a novel betacoronavirus strain, named Severe Acute Respiratory Syndrome-CoronaVirus 2 (SARS-CoV-2), and a fatal respiratory illness, COVID-19, formally recognised as a pandemic by the WHO has led to a rush by health systems all over the world to develop and implement testing for viral infection. The rapid cloning and sequencing of the viral genome permitted the development of PCR-based assays for the detection of viral nucleic acids that have become a key strategy for both clinical diagnosis and epidemiological monitoring studies. However, PCR testing is not 100% efficient, indeed, it has been reported that the overall positive rate of RNA testing can be as low as 30-50% in patients with COVID-19 (Liu et al. Positive rate of RT-PCR detection of SARS-CoV-2 infection in 4880 cases from one hospital in Wuhan, China, from Jan to Feb 2020. Clin Chim Acta. 2020;505:172-5; Yu et al. Quantitative Detection and Viral Load Analysis of SARS-CoV-2 in Infected Patients. Clin Infect Dis. 2020; Wang et al. The genetic sequence, origin, and diagnosis of SARS-CoV-2. Eur J Clin Microbiol Infect Dis. 2020). Further, testing for viral RNA requires specialised infrastructure and highly trained operators and, importantly, cannot detect evidence of past infection, which will be crucial for epidemiological efforts to assess how many people have been infected. Assays to measure antibody responses and determine seroconversion, while not appropriate to detect acute infections, are however, valuable sources of complementary information. Serological assays allow quantitative study of the immune response(s) to SARS-CoV-2 and are also critical for determination of the prevalence of infection in any given area; a necessary variable to define the infection fatality rate and that is of considerable utility for guiding management of the epidemic. Finally, quantitative and qualitative assays of antibody responses can aid in the identification of factors that correlate with effective immunity to SARS-CoV-2, the duration of these immune responses and may also aid in the selection of donors from whom preparations of convalescent serum/plasma can be generated for therapeutic use.

Multiple antibody tests to detect exposure to SARS-CoV-2, are becoming available, however the majority of these assays have been optimised to detect immunoglobulin G (IgG) antibodies to either the Spike (S) protein or the nucleoprotein of the virus. These proteins are key elements of the viral particle and are expected, by analogy with other coronaviruses, to be highly immunogenic. However, the immunogenicity of other viral proteins, 28 are encoded in the viral genome, has been little explored.

Here we have studied the antibody response to the main viral protease (Mpro, or 3CLPro) elicited after viral infection. Like other betacoronaviruses, SARS-CoV-2 is a positive-sense RNA virus that expresses all of its proteins as a single polypeptide chain. Mpro carries out the critical role in viral replication of cleaving the 1ab polyprotein to yield the mature proteins. Since this activity is essential for the viral life cycle, MPro structure and function has been studied intensively as specific inhibitors of this enzyme might act as potent anti-viral agents. We now report, for the first time, that individuals who have been infected with SARS-CoV-2 make high titre antibody responses to Mpro and that assay for seroreactivity to this protein sensitively and specifically discriminates between infected and non-infected individuals.

Thus, generally, the present invention provides for isolated and recombinantly expressed SARS-CoV-2 M^{pro} proteins, and fragments thereof, for the detection of SARS-CoV-2 specific antibodies in infected humans.

### Definitions

In the context of the present invention the 3C-like protease (3CLpro, also referred to as the main protease, M^{pro}) of SARS-CoV-2 is characterized by having residues 3264-3569 of the ORF1ab polyprotein of GenBank accession code MN908947.3. This amino acid sequence is also characterized herein as SEQ ID NO 1 (from hereinafter referred to as "SARS-CoV-2 M^{pro} protein".

A "fragment" of the SARS-CoV-2 M^{pro} protein according to the present invention is a partial amino acid sequence of the SARS-CoV-2 M^{pro} protein or a functional equivalent of such a fragment. A fragment is shorter than the complete SARS-CoV-2 M^{pro} protein and is preferably between about, 15, 20 or 65 and about 305 amino acids long, more preferably between about 15, 20 or 65 and about 250 amino acids long, even more preferably between about 65 and about 200 amino acids long. A fragment of the SARS-CoV-2 M^{pro} protein also includes peptides having at least 15, 20 or 65 contiguous amino acid residues having at least about 70%, at least about 80%, at least about 90%, preferably at least about 95%, more preferably at least 98% sequence identity with at least about 15, 20 or 65 contiguous amino acid residues of SEQ ID No. 1 having about the same length as said peptides. Depending on the expression system chosen, the protein fragments may or may not be expressed in native glycosylated form.

A fragment that "corresponds substantially to" a fragment of the SARS-CoV-2 M^{pro} protein is a fragment that has substantially the same amino acid sequence and has substantially the same functionality as the specified fragment of the SARS-CoV-2 M^{pro} protein.

A fragment that has "substantially the same amino acid sequence" as a fragment of the SARS-CoV-2 M^{pro} protein typically has more than 90% amino acid identity with this fragment. Included in this definition are conservative amino acid substitutions.

"Epitope" as used herein refers to an antigenic determinant of a polypeptide. An epitope could comprise three amino acids in a spatial conformation which is unique to the epitope. Generally, an epitope consists of at least five such amino acids, and more usually consists of at least 8-10 such amino acids. Methods of determining the spatial conformation of such amino acids are known in the art.

"Antibodies" as used herein are polyclonal and/or monoclonal antibodies or fragments thereof, including recombinant antibody fragments, as well as immunologic binding equivalents thereof, which are capable of specifically binding to the SARS-CoV-2 M^{pro} protein and/or to fragments thereof. The term "antibody" is used to refer to either a homogeneous molecular entity or a mixture such as a serum product made up of a plurality of different molecular entities. Recombinant antibody fragments may, e.g., be derived from a monoclonal antibody or may be isolated from libraries constructed from an immunized non-human animal.

"Sensitivity" as used herein in the context of testing a biological sample is the percentile of the number of true positive M^{pro} of SARS-CoV-2 samples divided by the total of the number of true positive M^{pro} of SARS-CoV-2 samples plus the number of false negative M^{pro} of SARS-CoV-2 samples.

"Specificity" as used herein in the context of testing a biological sample is the percentile of the number of true negative M^{pro} of SARS-CoV-2 samples divided by the total of the number of true negative M^{pro} of SARS-CoV-2 samples plus the number of false positive samples.

"Detection rate" as used herein in the context of antibodies specific for the SARS-CoV-2 virus is the percentile of the number of M^{pro} of SARS-CoV-2 positive samples in which the antibody was detected divided by the total number of M^{pro} of SARS-CoV-2 positive samples tested. "Overall detection rate" as used herein refers to the virus detection obtained by detecting both IgM and IgG.

A "clinical sample" comprises biological samples from one or from a random mix of patients, including patients with and without SARS-CoV-2.

"Onset of symptoms" as used herein is the onset of fever and a cough.

The terms "sequence identity" or "percent identity" in the context of two or more polypeptides or proteins refers to two or more sequences or subsequences that are the same ("identical") or have a specified percentage of amino acid residues that are identical ("percent identity") when compared and aligned for maximum correspondence with a second molecule, as measured using a sequence comparison algorithm (e.g., by a BLAST alignment, or any other algorithm known to persons of skill), or alternatively, by visual inspection.

A protein or peptide of the present invention has substantial identity with another if, optimally aligned, there is an amino acid sequence identity of at least about 60% identity with a naturally-occurring protein or with a peptide derived therefrom, usually at least about 70% identity, more usually at least about 80% identity, preferably at least about 90% identity, and more preferably at least about 95% identity, and most preferably at least about 98% identity. Identity means the degree of sequence relatedness between two polypeptide or two polynucleotides sequences as determined by the identity of the match between two strings of such sequences, such as the full and complete sequence. Identity can be readily calculated. While there exist a number of methods to measure identity between polypeptide sequences, the term "identity" is well known to skilled artisans.

### Description

In a first aspect of the present invention, we herein provide an *in vitro* method for detecting in at least one biological sample an antibody that binds to at least one epitope of the SARS-CoV-2 virus, comprising:
a. contacting said at least one biological sample with at least one isolated SARS-CoV-2 M^{pro} protein, or at least one fragment of said isolated SARS-CoV-2 M^{pro} protein comprising at least one epitope of the SARS-CoV-2 virus, and
b. detecting the formation of an antigen-antibody complex between said virus protein or said fragment and an antibody present in said biological sample.

It is herein noted, that to produce high amounts of the at least one isolated SARS-CoV-2 M^{pro} protein or at least one fragment of said isolated SARS-CoV-2 M^{pro} protein comprising at least one epitope of the SARS-CoV-2 virus thereof, the DNA fragments from genomic RNA can be produced by RT-PCR. The appropriate PCR primers can include restriction enzyme cleavage sites. After purification, the PCR products can be digested with the suitable restriction enzymes and cloned into suitable expression vectors, preferably, under the control of a strong promotor. The vectors then can be transformed into an appropriate host cell. Positive clones can be identified by PCR screening and further confirmed by enzymatic cut and sequence analysis. The so produced proteins/fragments then can be tested for their suitability as antigens for the method of the first aspect.

In a preferred embodiment of the first aspect, said at least one isolated SARS-CoV-2 M^{pro} protein is the protein of SEQ ID NO 1 or a variant of SEQ ID NO 1 having at least 80%, 85%, 90% or 95% sequence identity to SEQ ID NO 1, e.g., 95%, 96%, 97%, 98%, or 99%, sequence identity to SEQ ID NO 1.

In another preferred embodiment of the first aspect, said at least one fragment of said isolated SARS-CoV-2 M^{pro} protein comprising at least one epitope of the SARS-CoV-2 virus, comprises at least 15, 20 or 65 contiguous amino acid residues having at least about 70%, at least about 80%, at least about 90%, preferably at least about 95%, more preferably at least 98% sequence identity with at least about 15, 20 or 65 contiguous amino acid residues of SEQ ID No. 1. Preferably, said at least one fragment of said isolated SARS-CoV-2 M^{pro} protein has a sensitivity of more than about 70%, at least about 80%, at least about 90%, preferably at least about 95%, more preferably at least 98% or 99%. Preferably, said at least one fragment of said isolated SARS-CoV-2 M^{pro} protein has a specificity of more than about 70%, at least about 80%, at least about 90%, preferably at least about 95%, more preferably at least 98% or 99%. More preferably, said at least one fragment of said isolated SARS-CoV-2 M^{pro} protein has a detection rate or an overall detection rate of more than about 65%, at least about 70%, at least about 80%, at least about 90%, preferably at least about 95%, more preferably at least 98% or 99%.

In still another preferred embodiment of the first aspect or of any of its preferred embodiments, said method is adapted to detect IgG, IgM and/or IgA. Preferably, said method is adapted to detect IgG. In another preferred embodiment said method is adapted to detect IgG at a dilution of a fluid biological sample, preferably serum, of about 1 :100, about 1 :800, about 1 :900, about 1 :1000, about 1 :1100 up to about 1 :1200, 1:800 or 1:3200. In another preferred embodiment, the method according to the present invention is able to detect IgM at a dilution of a fluid biological sample, preferably serum, of about 1 :50, about 1 :100, about 1 :200 up to about 1:400, or 1:1000.

In another preferred embodiment of the first aspect or of any of its preferred embodiments, said at least one isolated SARS-CoV-2 M^{pro} protein or fragment thereof is a recombinant expression product.

In still another preferred embodiment of the first aspect or of any of its preferred embodiments, appropriate biological samples to practice the method include, but are not limited to, mouth gargles, any biological fluids, virus isolates, tissue sections, wild and laboratory animal samples. Preferably, said biological sample is a blood, plasma or serum sample isolated from a subject, preferably a mammal, more preferably from a human being. Preferably, said biological sample is a serum or sera sample.

In still another preferred embodiment of the first aspect or of any of its preferred embodiments, said method is an *in vitro* diagnostic method for the detection of a subject having antibodies against the SARS-CoV-2 virus, wherein said subject is preferably a mammal, more preferably a human being, and wherein said subject is diagnosed as having antibodies against the SARS-CoV-2 virus if an antigen-antibody complex between said virus protein or said fragment and an antibody present in said biological sample is detected.

In a preferred embodiment, said *in vitro* diagnostic method according to the present invention, will be able to additionally detect a wide array of stages of a SARS-CoV-2 infection. In still another preferred embodiment, said diagnostic method will be able to detect early stages of a SARS-CoV-2 infection. In another preferred embodiment, said diagnostic method will be able to detect early stages of infection by being able to detect IgM. In another preferred embodiment, said diagnostic method will be able to detect later stages of infection or a past infection by being able to detect IgG. In another preferred embodiment, said diagnostic method will be able to detect early stages of infection by being able to detect very low concentrations of antibodies. Accordingly, in a preferred embodiment the diagnostic method is adapted to detect antibodies against a SARS-CoV-2 virus less than about 50 days after the onset of symptoms, preferably less than about 40, less than about 30, less than about 25, less than about 20, less than about 15, less than about 12, less than about 10, less than about 9, less than about 8, less than about 7, less than about 6, less than about 5 less, or than about 4 days after the onset of symptoms.

In still another preferred embodiment of the first aspect or of any of its preferred embodiments, said method is an *in vitro* method for screening individuals having antibodies against the SARS-CoV-2 virus from those not having antibodies against the SARS-CoV-2 virus.

In still another preferred embodiment of the first aspect or of any of its preferred embodiments, the existence of antigen-antibody binding can be detected via methods well known in the art. In western blotting, one preferred method according to the present invention, fragments of a protein are transferred from the gel to a stable support such as a nitrocellulose membrane. The protein fragments can be reacted with sera from individuals suspected of having been infected with the SARS-CoV-2 virus. This step is followed by a washing step that will remove unbound antibody but retains antigen-antibody complexes. The antigen-antibody complexes then can be detected via anti-immunoglobulin antibodies which are labelled, e.g., with radioisotopes. Use of a western blot thus allows detection of the binding of sera of SARS-CoV-2 positive human to the M^{pro} of SARS-CoV-2 protein or a fragment thereof.

Other preferred detections methods include enzyme-linked immunosorbent assays (ELISA) and dot blotting. Both of these methods are relatively easy to use and are high throughput methods. ELISA, in particular, has achieved high acceptability with clinical personnel. ELISA, preferably based in chemiluminescent or colorimetric methods, is also highly sensitive. However, any other suitable method to detect antigen-antibody complexes such as, but not limited to, standardized radio immunoassays (RIA), lateral flow tests, also known as lateral flow immunochromatographic assays, or immunofluorescence assays (IFA), also can be used. Preferably, a specially preferred method is the ELISA assay, more preferably a chemiluminescent enzyme linked immunosorbent assay (ELISA).

In still another preferred embodiment of the first aspect or of any of its preferred embodiments, the method of detection is an ELISA assay by using the ELISA system as described later in the present specification.

In still another preferred embodiment of the first aspect or of any of its preferred embodiments, said biological sample is contacted with at least one or more further SARS-CoV-2 immunogens or fragments thereof, wherein preferably said immunogens are selected from the group consisting of nucleocapsid (N) proteins of SARS-CoV-2, and spike (S) domains including the S1 subunit, and/or receptor binding domain (RBD) of SARS-CoV-2.

In still another preferred embodiment of the first aspect or of any of its preferred embodiments, said biological sample is contacted with at least one or more further immunogens derived from at least one distinct isolated SARS protein.

A second aspect of the invention refers to an *in vitro* kit for detecting in a biological sample an antibody that binds to at least one epitope of the SARS-CoV-2 virus comprising:
a. at least one isolated SARS-CoV-2 M^{pro} protein, or at least one fragment of said isolated SARS-CoV-2 M^{pro} protein comprising at least one epitope of the SARS-CoV-2 virus, and
b. reagents for detecting the formation of antigen-antibody complex between said at least one isolated SARS-CoV-2 M^{pro} protein or a fragment thereof and at least one antibody present in a biological sample,
wherein said at least one isolated protein or fragment thereof and said reagents are present in an amount sufficient to detect the formation of said antigen-antibody complex.

In a preferred embodiment of the second aspect of the invention, said at least one isolated SARS-CoV-2 M^{pro} protein is the protein of SEQ ID NO 1 or a variant of SEQ ID NO 1 having at least 80%, 85%, 90% or 95% sequence identity to SEQ ID NO 1, e.g., 95%, 96%, 97%, 98%, or 99%, sequence identity to SEQ ID NO 1.

In another preferred embodiment of the second aspect of the invention, said at least one fragment of said isolated SARS-CoV-2 M^{pro} protein comprising at least one epitope of the SARS-CoV-2 virus, comprises at least 15, 20 or 65 contiguous amino acid residues having at least about 70%, at least about 80%, at least about 90%, preferably at least about 95%, more preferably at least 98% sequence identity with at least about 15, 20 or 65 contiguous amino acid residues of SEQ ID No. 1.

In another preferred embodiment of the second aspect of the invention, said reagents are capable of detecting IgG, IgM and/or IgA. Preferably, said reagents are capable of detecting IgG. In another preferred embodiment said reagents are capable of detecting IgG at a dilution of about 1 :100, about 1 :800, about 1 :900, about 1 :1000, about 1 :1100 up to about 1 :1200. In another preferred embodiment, said reagents are capable of detecting IgM at a dilution of about 1 :50, about 1 :100, about 1 :500 up to about 1 :1000.

In a particularly preferred embodiment, the kit is suitable for performing a radioimmunoassay (RIA), enzyme linked immunosorbent assay (ELISA) preferably a chemiluminescent or colorimetric enzyme linked immunosorbent assay (ELISA), immunofluorescence assay (IFA), dot blot, lateral flow test, also known as lateral flow immunochromatographic assay, or western blot. Preferably, the kit is an ELISA system.

In the context of the present invention, the ELISA (enzyme-linked immunosorbent assay) system is preferably understood as a plate-based assay technique designed for detecting and quantifying at least one isolated SARS-CoV-2 M^{pro} protein or fragment thereof. In this ELISA, the at least one isolated SARS-CoV-2 M^{pro} protein or fragment thereof must be immobilized to a solid surface and then exposed to the biological sample to form a complex. Detection is accomplished by any techniques well known in the art.

ELISAs, according to the present invention, are typically performed in 96-well (or 384-well) polystyrene plates, which will passively bind the at least one isolated SARS-CoV-2 M^{pro} protein or fragments thereof. The binding and immobilization of reagents makes ELISAs simple to design and perform. Having the reactants of the ELISA immobilized to the microplate surface enables easy separation of bound from non-bound material during the assay. This ability to wash away non-specifically bound materials makes the ELISA a powerful tool for measuring specific analytes within a crude preparation.

In a preferred embodiment of the second aspect of the invention the ELISA system comprises at least one isolated SARS-CoV-2 M^{pro} protein or fragments thereof to coat or coating a solid surface, preferably microtiter plate wells, and optionally one or more of the following reagents: blocking reagents for unbound sites to prevent false positive results; anti-(species) IgG, IgM and/or IgA conjugated to a label, preferably an enzyme; and substrates that react with the label, preferably the enzyme, to indicate a positive reaction. In addition to the procedure reagents, additional reagents such as wash buffers, stop solutions and stabilizers can enhance the quality of the ELISA assay. When choosing individual reagents, or complete kits, it is helpful to know the sensitivity required and whether one is trying to detect an analyte or the antibody response to it.

In still another preferred embodiment of the second aspect of the invention, said at least one isolated SARS-CoV-2 M^{pro} protein or fragment thereof is a recombinant expression product.

In still another preferred embodiment of the second aspect of the invention, said at least one isolated SARS-CoV-2 M^{pro} protein is the protein of SEQ ID NO 1.

A third aspect of the invention refers to the use of kit of the second aspect of the invention or of any of its preferred embodiments, for implementing any of the methods disclosed in the first aspect of the invention.

The uses of the at least one isolated SARS-CoV-2 M^{pro} protein, or at least one fragment of said isolated SARS-CoV-2 M^{pro} protein comprising at least one epitope of the SARS-CoV-2 virus, according to the present invention that are described above are those which presently appear most attractive. However, the foregoing disclosures of embodiments of the invention and uses therefor have been given merely for purposes of illustration and not to limit the invention. Thus, the invention should be considered to include all embodiments falling within the scope of the claims following the Example section and any equivalents thereof.

The following examples are merely illustrative and should not be construed to limit in any way the invention as set forth in the claims which follow.

### Examples

The following sequences were used to carried out the examples and figures illustrated in the present description and figures:

### Sequence listing

Peptide sequence SARS-CoV-2 Mpro (SEQ ID NO 1): 
SAVLQSGFRKMAFPSGKVEGCMVQVTCGTTTLNGLWLDDVVYCPRHVICTSEDMLNP
NYEDLLIRKSNHNFLVQAGNVQLRVIGHSMQNCVLKLKVDTANPKTPKYKFVRIQPGQ
TFSVLACYNGSPSGVYQCAMRPNFTIKGSFLNGSCGSVGFNIDYDCVSFCYMHHMEL
PTGVHAGTDLEGNFYGPFVDRQTAQAAGTDTTITVNVLAWLYAAVINGDRWFLNRFTT
TLNDFNLVAMKYNYEPLTQDHVDILGPLSAQTGIAVLDMCASLKELLQNGMNGRTILGS
ALLEDEFTPFDVVRQCSGVTFQLEHHHHHH
Nucleotide sequence of SARS-CoV-2 Mpro (SEQ ID NO 2): 
tcagctgttttgcagagtggttttagaaaaatggcattcccatctggtaaagttgagggttgtatggtacaagtaacttgtggta
caactacacttaacggtctttggcttgatgacgtagtttactgtccaagacatgtgatctgcacctctgaagacatgcttaacc
ctaattatgaagatttactcattcgtaagtctaatcataatttcttggtacaggctggtaatgttcaactcagggttattggacattc
tatgcaaaattgtgtacttaagcttaaggttgatacagccaatcctaagacacctaagtataagtttgttcgcattcaaccagg
acagactttttcagtgttagcttgttacaatggttcaccatctggtgtttaccaatgtgctatgaggcccaatttcactattaaggg
ttcattccttaatggttcatgtggtagtgttggttttaacatagattatgactgtgtctctttttgttacatgcaccatatggaattacca
actggagttcatgctggcacagacttagaaggtaacttttatggaccttttgttgacaggcaaacagcacaagcagctggta
cggacacaactattacagttaatgttttagcttggttgtacgctgctgttataaatggagacaggtggtttctcaatcgatttacc
acaactcttaatgactttaaccttgtggctatgaagtacaattatgaacctctaacacaagaccatgttgacatactaggacct
ctttctgctcaaactggaattgccgttttagatatgtgtgcttcattaaaagaattactgcaaaatggtatgaatggacgtaccat
attgggtagtgctttattagaagatgaatttacaccttttgatgttgttagacaatgctcaggtgttactttccaactcgagcacca
ccaccaccaccactga
Peptide sequence SARS-CoV-2 nucleocapsid protein (SEQ ID NO 3): 
MASDNGPQNQRNAPRITFGGPSDSTGSNQNGERSGARSKQRRPQGLPNNTASWFT
ALTQHGKEDLKFPRGQGVPINTNSSPDDQIGYYRRATRRIRGGDGKMKDLSPRWYFY
YLGTGPEAGLPYGANKDGIIWVATEGALNTPKDHIGTRNPANNAAIVLQLPQGTTLPKG
FYAEGSRGGSQASSRSSSRSRNSSRNSTPGSSRGTSPARMAGNGGDAALALLLLDR
LNQLESKMSGKGQQQQGQTVTKKSAAEASKKPRQKRTATKAYNVTQAFGRRGPEQT
QGNFGDQELIRQGTDYKHWPQIAQFAPSASAFFGMSRIGMEVTPSGTWLTYTGAIKLD
DKDPNFKDQVILLNKHIDAYKTFPPTEPVEHHHHHH
Nucleotide sequence of SARS-CoV-2 nucleocapsid protein (SEQ ID NO 4): 
atggcttctgataatggtccgcaaaatcagcgtaatgcaccccgcattacgtttggtggaccctcagattcaactggcagta
accagaatggagaacgcagtggggcgcgatcaaaacaacgtcggccccaaggtttacccaataatactgcgtcttggtt
caccgctctcactcaacatggcaaggaagaccttaaattccctcgaggacaaggcgttccaattaacaccaatagcagtc
cagatgaccaaattggctactaccgaagagctaccagacgaattcgtggtggtgacggtaaaatgaaagatctcagtcc
aagatggtatttctactacctaggaactgggccagaagctggacttccctatggtgctaacaaagacggcatcatatgggtt
gcaactgagggagccttgaatacaccaaaagatcacattggcacccgcaatcctgctaacaatgctgcaatcgtgctac
aacttcctcaaggaacaacattgccaaaaggcttctacgcagaagggagcagaggcggcagtcaagcctcttctcgttc
ctcatcacgtagtcgcaacagttcaagaaattcaactccaggcagcagtaggggaacttctcctgctagaatggctggca
atggcggtgatgctgctcttgctttgctgctgcttgacagattgaaccagcttgagagcaaaatgtctggtaaaggccaaca
acaacaaggccaaactgtcactaagaaatctgctgctgaggcttctaagaagcctcggcaaaaacgtactgccactaaa
gcatacaatgtaacacaagctttcggcagacgtggtccagaacaaacccaaggaaattttggggaccaggaactaatc
agacaaggaactgattacaaacattggccgcaaattgcacaatttgcccccagcgcttcagcgttcttcggaatgtcgcgc
attggcatggaagtcacaccttcgggaacgtggttgacctacacaggtgccatcaaattggatgacaaagatccaaatttc
aaagatcaagtcattttgctgaataagcatattgacgcatacaaaacattcccaccaacagagcctgtcgagcaccacca
ccaccaccactga
Peptide sequence SARS-CoV-2 RBD (receptor binding domain) (SEQ ID NO 5): 
gsrNLCPFGEVFNATRFASVYAWNRKRISNCVADYSVLYNSASFSTFKCYGVSPTKLND
LCFTNVYADSFVIRGDEVRQIAPGQTGKIADYNYKLPDDFTGCVIAWNSNNLDSKVGG
NYNYLYRLFRKSNLKPFERDISTEIYQAGSTPCNGVEGFNCYFPLQSYGFQPTNGVGY
QPYRVVVLSFELLHAPATVCGPKlvpr
Nucleotide sequence of SARS-CoV-2 RBD (SEQ ID NO 6):

ggatcccgcaacttgtgcccttttggtgaagtttttaacgccaccagatttgcatctgtttatgcttggaacaggaagagaatc
agcaactgtgttgctgattattctgtcctatataattccgcatcattttccacttttaagtgttatggagtgtctcctactaaattaaat
gatctctgctttactaatgtctatgcagattcatttgtaattagaggtgatgaagtcagacaaatcgctccagggcaaactgga

aagattgctgattataattataaattaccagatgattttacaggctgcgttatagcttggaattctaacaatcttgattctaaggtt
ggtggtaattataattacctgtatagattgtttaggaagtctaatctcaaacctttgagagagatatttcaactgaaatctatca
ggccggtagcacaccttgtaatggtgttgaaggttttaattgttactttcctttacaatcatatggtttccaacccactaatggtgtt
ggttaccaaccatacagagtagtagtactttcttttgaacttctacatgcaccagcaactgtttgtggacctaaacttgtccctc
gc

### Materials and methods

### Molecular cloning of the SARS-CoV-2 Cys-like protease (3CLpro, Mpro) and nucleocapsid proteins (NP).

A gene encoding SARS-CoV-2 Mpro (ORF1ab polyprotein residues 3264-3569, GenBank code:MN908947.3) was amplified by PCR using the oligos 5'-gacccatggcttcagctgtttttcagagtggttt-3' and 5'-gacctcgagttggaaagtaacacctgagcatt-3', digested with Ncol and Xhol and ligated into the vector pET22b (Novagen) digested with the same restriction enzymes. The integrity of this construct was verified by sequencing at MWG Eurofins.

Oligonucleotides 5'-gatccatggcttctgataatggtccgcaaaatcagcgtaatgca-3' and 5'-caggtcgacaggctctgttggtgggaatg-3'were used to amplify the nucleocapsid protein (NP) of SARS-CoV-2. The amplification product was then digested with Ncol and Sall and ligated into the pET26b vector (Novagen) digested with Ncol and Xhol. The integrity of this construct was verified by sequencing at MWG Eurofins.

### Expression of the SARS-CoV-2 Cys-like protease (3CLpro, Mpro) and nucleocapsid proteins

SARS-CoV-2 Mpro protein was expressed by transforming this plasmid into the E. coli strain BL21 Star (DE3) pLysS (ThermoFisher). Transformed clones were pre-cultured overnight at room temperature in 50 mL 1 x LB medium with ampicillin (150 µg/mL) and chloramphenicol (34ug/ml). The overnight culture was then inoculated into 1L of 1 x LB medium (150 µg/mL ampicillin and 34ug/ml chloramphenicol) and the culture was grown at 37°C with agitation until the OD₆₀₀ reached 0.6 when Isopropyl-D-thiogalactoside (IPTG) was added to 1mM to induce overexpression of the Mpro gene. The same protocol was followed to produce nucleocapsid protein except that kanamycin (150ug/ml) was used instead of ampicillin for antibiotic-mediated selection.

After overnight culture at 22°C, bacteria were harvested by centrifugation at 9500 x g, 4°C for 15 min and the pellets were washed by resuspension in 150 mL TES buffer (20 mM Tris pH 8, 2mM EDTA, 150 mM NaCI) and re-centrifugation. Washed pellets were either processed immediately or stored frozen for later use.

Fresh, or thawed, cell pellets were resuspended in ice-cold 50 mM NaH₂PO₄ buffer pH8, 500 mM NaCl, 10 mM imidazole (I2399, Sigma Aldrich), 0.1% Sarkosyl, and 5% glycerol (pH 8.0). Lysozyme was then added (to 0.25 mg/ml) as were phenylmethylsulfonyl fluoride, Leupeptin and Pepstatin A (all to a final concentration of 1mM) and DNase I (2 µg/ml). Bacteria were lysed by sonication (3 cycles of 30 seconds with 30 seconds rest on ice between pulses) and soluble proteins were separated by centrifugation of the lysed cells at 14,000g at 4 °C for 45 minutes.

6-histidine tagged proteins were purified from the lysate using 5-ml HiTrap Ni2+ chelating columns (Agarose bead technologies). The bacterial supernatant was loaded on the column at a flow rate of 1 ml/min, followed by washing with 5 column volumes of 50 mM NaH₂PO₄ buffer, 500 mM NaCl, 10 mM imidazole and then 5 column volumes of 50 mM NaH₂PO₄ buffer, 500 mM NaCl, 25 mM imidazole. Recombinant proteins were eluted using a linear gradient of imidazole ranging from 25 mM to 250 mM over 5 column volumes. The proteins were then further purified by gel filtration using a 10/30 Superdex 75 Increase column (Cytiva) pre-equilibrated in 10mM Tris, 2mM EDTA, 300mM NaCl, pH 7.5. The gel filtration analysis indicated that the SARS CoV 2 Mpro protein purified as a dimer.

### Molecular cloning and production of SARS-CoV-2 Receptor Binding Domain protein in mammalian cells (mRBD).

The cDNA region coding for the Receptor Binding Domain (RBD) (residues 334-528) defined in the structure of the S protein (PDB ID 6VSB) was amplified for expression in mammalian cells. The fragment was cloned in frame with the IgK leader sequence, an HA-tag (YPYDVPDYA) and a thrombin recognition site (LVPRGS) at its 5' end, and it was followed by a second thrombin site, the TIM-1 mucin domain and the human IgG1 Fc region at the 3' end. The recombinant cDNA was cloned in a vector derived from the pEF-BOS (9) for transient expression in HEK293 cells, and in the pBJ5-GS vector for stable protein production in CHO cells following the glutamine synthetase system (Casasnovas JM, and Springer TA. Kinetics and thermodynamics of virus binding to receptor. Studies with rhinovirus, intercellular adhesion molecule-1 (ICAM-1), and surface plasmon resonance. The Journal of biological chemistry. 1995;270(22):13216-24). The inclusion of the TIM-1 mucin domain enhanced protein expression.

Mammalian RBD (mRBD) fused to the mucin domain and the Fc region (mRBD-mucin-Fc) was initially purified from cell supernatants by affinity chromatography using an IgSelect column (GE Healthcare). The mucin-Fc portion and the HA-tag were released from the mRBD protein by overnight treatment with thrombin at RT. The mixture was run through a protein A column to remove the mucin-Fc protein and mRBD was further purified by size-exclusion chromatography with a Superdex 75 column in HBS buffer (25 mM HEPES and 150 mM NaCl, pH 7.5). The concentration of purified mRBD was determined by absorbance at 280 nm.

### ELISA for detection of antibodies to SARS-CoV-2

96-well Maxisorp Nunc-Immuno plates were coated with 100 µL/well of recombinant proteins diluted in borate buffered saline (BBS, 10 mM borate, 150 mM NaCl, pH 8.2); NP and the protease at 0.5 µg/ml, RBD at 1µg/ml and incubated overnight at 4°C. Coating solutions were then aspirated, the ELISA plates were washed three times with 200 µl of PBS 0.05% Tween 20 (PBS-T) and then dried before blocking with PBS with 1% casein (Biorad) for 2 hours at room temperature. The plates were washed again with PBS-T and 100 µl of patient serum/plasma sample diluted in PBS-casein, 0.02% Tween-20, as indicated, was added and incubated for 2 hours at 37°C. The plates were washed again and 100 µL/well of the indicated detection antibody, all from Jackson Labs (AffiniPure Rabbit Anti-Human IgM, Fcµ fragment specific. HRPO, AffiniPure Rabbit Anti-Human Serum IgA, α chain specific. HRPO, or AffiniPure Rabbit Anti-Human IgG, Fcγ fragment specific. HRPO) was added and incubated for 1 hour at room temperature. The plates were washed with PBS-T four times and incubated at room temperature in the dark with 100 µL/well of Substrate Solution (OPD, Sigma prepared according to the manusfacturer's instructions) (typically for 3 minutes). 50 µL of stop solution (3M H₂SO₄) were then added to each well and the optical density (at 492nm) of each well was determined using a microplate reader.

Negative controls included wells coated just with blocking buffer and serum samples collected from donors before 2019.

### Results

### Production of soluble Cys-like protease (3CLpro, Mpro) from SARS-CoV-2.

To explore the similarity between the Cys-like proteases of different coronaviruses, 3CLpro (Mpro) from SARS-CoV-2, HCovNL63 and HCov229E were aligned (Figure 6). The degree of similarity was around 40%.

The nucleotide sequence (SEQ ID NO 2) from the SARS-CoV-2 Cys-like protease (also known as 3CLpro, MPro) from the Wuhan-Hu-1 strain (GenBank accession number MN908947.3) was amplified and subcloned into the prokaryotic expression vector pET22b and the soluble protein was expressed in the E. *coli* strain BL21 Star (DE3) pLysS. After extraction of the soluble proteins from the bacterial lysates and selection of the His-tagged proteins in a Ni-NTA column, Mpro was purified by size exclusion yielding a protein with an apparent Mw of around 70kDa, corresponding to a dimer. Figure 1 shows schematic representations and SDS-PAGE data of the recombinant proteins.

Since the aim was to evaluate, for the first time, if coronavirus-infected individuals could generate an antibody response against Cys-like proteases, other SARS-CoV-2 proteins were also produced: NP (SEQ ID NO 4) was expressed in *E coli,* and RBD was expressed by transfection in mammalian cells (mRBD of SEQ ID NO 5). NP and protease had a Histidine tag and they were purified on Ni²⁺-NTA columns followed by size exclusion.

### High titres of SARS-CoV-2 3CLpro-specific antibodies can be detected in Covid-19 positive patient sera but not in negative donors.

First experiments using covid-19 patient sera revealed the presence of MPro antibodies at high titres. Antibody reactivity to the protease reached saturation at low concentrations and discriminated efficiently between individuals who had been infected with SARS-CoV-2 and those that did not have the disease (Figure 2A). Further, it was possible to detect antibodies of the three isotypes, IgG, IgM and IgA. The reactivity of the different sera against the protease MPro was comparable or in certain cases stronger to the reactivity against the other viral protein (Figure 2B). The specificity of the recognition was obvious in coating titration experiments and sera titration (Figure 4).

### Specificity and sensitivity / ROC analysis of above data

ELISAs based on both antigens (NP and protease-His) detect IgG antibodies with high sensitivity and specificity, the assay based on the protease as antigen at least as sensitive and specific as NP (Figure 3).

The comparison, as shown in figure 5, using serum samples, of ELISAs based on the nucleoprotein, protease and mRBD antigens, suggests that assays using the N and P antigens may be more sensitive than those using mRBD.

## Claims

1. An *in vitro* method for detecting in at least one biological sample an antibody that binds to at least one epitope of the SARS-CoV-2 virus, comprising:
a. contacting said at least one biological sample with at least one isolated SARS-CoV-2 M^{pro} protein, or at least one fragment of said isolated SARS-CoV-2 M^{pro} protein comprising at least one epitope of the SARS-CoV-2 virus, and
b. detecting the formation of an antigen-antibody complex between said virus protein or said fragment and an antibody present in said biological sample.

2. The *in vitro* method of claim 1, wherein said at least one isolated SARS-CoV-2 M^{pro} protein is the protein of SEQ ID NO 1 or a variant of SEQ ID NO 1 having at least 80% sequence identity to SEQ ID NO 1.

3. The *in vitro* method of claim 1, wherein said at least one fragment of said isolated SARS-CoV-2 M^{pro} protein comprising at least one epitope of the SARS-CoV-2 virus, comprises at least 20 contiguous amino acid residues having at least 80% sequence identity with at least about 20 contiguous amino acid residues of SEQ ID No. 1.

4. The *in vitro* method of claim 1, wherein said at least one isolated SARS-CoV-2 M^{pro} protein is the protein of SEQ ID NO 1.

5. The *in vitro* method of any of claims 1 to 4, wherein said at least one isolated SARS-CoV-2 M^{pro} protein or fragment thereof is a recombinant expression product.

6. The *in vitro* method of any of claims 1 to 5, wherein said biological sample is a blood, plasma or serum sample.

7. The *in vitro* method of claim 6, wherein said biological sample is a serum sample, and said SARS-CoV-2 M^{pro} protein is the protein of SEQ ID NO 1.

8. The *in vitro* method of any of claims 1 to 7, wherein said method is an *in vitro* diagnostic method for the detection of a subject having antibodies against the SARS-CoV-2 virus, wherein said subject is diagnosed as having antibodies against the SARS-CoV-2 virus if an antigen-antibody complex between said virus protein, or said fragment, and an antibody present in said biological sample is detected.

9. The *in vitro* method of any of claims 1 to 7, wherein said method is a method for screening individuals having antibodies against the SARS-CoV-2 virus from those not having antibodies against the SARS-CoV-2 virus.

10. An *in vitro* kit suitable for detecting in a biological sample an antibody that binds to at least one epitope of the SARS-CoV-2 virus comprising:
a. at least one isolated SARS-CoV-2 M^{pro} protein, or at least one fragment of said isolated SARS-CoV-2 M^{pro} protein comprising at least one epitope of the SARS-CoV-2 virus, and
b. reagents for detecting the formation of antigen-antibody complex between said at least one isolated SARS-CoV-2 M^{pro} protein, or a fragment thereof, and at least one antibody present in a biological sample,
wherein said at least one isolated protein or fragment thereof and said reagents are present in an amount sufficient to detect the formation of said antigen-antibody complex.

11. The kit of claim 10, wherein said at least one isolated SARS-CoV-2 M^{pro} protein is the protein of SEQ ID NO 1 or a variant of SEQ ID NO 1 having at least 80% sequence identity to SEQ ID NO 1.

12. The kit of claim 10, wherein said at least one fragment of said isolated SARS-CoV-2 M^{pro} protein comprising at least one epitope of the SARS-CoV-2 virus, is **characterized by** comprising at least 20 contiguous amino acid residues having at least 80% sequence identity with at least 20 contiguous amino acid residues of SEQ ID No. 1.

13. The kit of any of claims 10 to 12, wherein said at least one isolated SARS-CoV-2 M^{pro} protein or fragment thereof is a recombinant expression product.

14. The kit of any of claims 10 to 13, wherein said at least one isolated SARS-CoV-2 M^{pro} protein is the protein of SEQ ID NO 1.

15. The kit of any of claims 10 to 14, wherein said kit is an ELISA system comprising at least one isolated SARS-CoV-2 M^{pro} protein of SEQ ID NO 1, to coat or coating a solid surface, preferably microtiter plate wells, and one or more of the following reagents: blocking reagents for unbound sites to prevent false positive results; anti-(species) IgG, IgM and/or IgA conjugated to a label, preferably an enzyme; and substrates that react with the label, preferably the enzyme, to indicate a positive reaction.

16. The in vitro method of any of claims 1 to 9, wherein the formation of antigen-antibody complex is detected by radioimmunoassay (RIA), enzyme linked immunosorbent assay (ELISA), chemiluminescent or colorimetric enzyme linked immunosorbent assay (ELISA), immunofluorescence assay (IFA), dot blot, a lateral flow immunochromatographic assay or western blot.

17. The in vitro method of any of claims 1 to 9, wherein the formation of antigen-antibody complex is detected by enzyme linked immunosorbent assay (ELISA).

18. The in vitro method of any of claims 1 to 9 or 16 to 17, wherein said method is capable of detecting IgG, IgM and/or IgA.

19. The in vitro method of any of claims 1 to 9 or 16 to 17, wherein said method is capable of detecting IgG.

20. The in vitro method of any of claims 1 to 9 or 16 to 17, wherein said method is capable of detecting IgM.

21. The in vitro method of any of claims 1 to 9 or 16 to 17, wherein said method is capable of detecting IgA.

22. The in vitro method of any of claims 1 to 9 or 16 to 21, wherein the formation of antigen-antibody complex is detected by any of the methods identified in claim 16 and said at least one virus protein or said fragment is adapted to detect IgG, IgM and/or IgA at a dilution of between 1:200 to 1:1800.

23. The in vitro method of any of claims 1 to 9 or 16 to 21, wherein the formation of antigen-antibody complex is detected by any of the methods identified in claim 16 and said is capable of detecting IgG, IgM and/or IgA at a dilution of between 1:200 to 1:1800.

24. The in vitro method of any of claims 1 to 9 or 16 to 23, wherein said biological sample is contacted with at least one or more further SARS-CoV-2 M^{pro} immunogens or fragments thereof.

25. The in vitro method of claim 24, wherein said further immunogens are selected from the group consisting of nucleocapsid (N) proteins of SARS-CoV-2, and spike (S) domains including the S1 subunit, and/or receptor binding domain (RBD) of SARS-CoV-2.

26. The in vitro method of any of claims 1 to 9 or 16 to 25, wherein said biological sample is contacted with at least one or more further immunogens derived from at least one distinct isolated SARS protein.

27. An ELISA system comprising at least one isolated SARS-CoV-2 M^{pro} protein or fragments thereof, as defined in any of claims 1 to 6, to coat or coating a solid surface, preferably microtiter plate wells, and one or more of the following reagents: blocking reagents for unbound sites to prevent false positive results; anti-(species) IgG, IgM and/or IgA conjugated to a label, preferably an enzyme; and substrates that react with the label, preferably the enzyme, to indicate a positive reaction.

28. The ELISA system of claim 27, wherein it further comprises additional reagents such as wash buffers, stop solutions and stabilizers.

29. In vitro use of the kit of any of claims 10 to 15 or the ELISA system of any of claims 27 or 28, for use in the implementation of the method of any of claims 1 to 9 or 16 to 26.
